# DEMANDE DE BREVET EUROPEEN

(11) **EP 2 198 850 A1**
(43) Date de publication de la demande: **23.06.2010**
(21) Numéro de dépôt: 09306274.3
(22) Date de dépôt: 18.12.2009
(51) Int. Cl.: A61K 8/39, A61K 8/44, A61K 8/46, A61K 8/60, A61K 8/92, A61Q 5/02, A61Q 5/12

(54) **Composition cosmétique détergente comprenant quatre tensioactifs et un corps gras non siliconé**

(30) Priorité: 22.12.2008 FR 0858965; 22.12.2008 FR 0858963
(71) Demandeur: L'Oréal, 75008 Paris (FR)
(72) Inventeur: Fack, Géraldine, 92300 Levallois-Perret (FR)
(74) Mandataire: Dossmann, Gérard

(57) **Abrégé**

L'invention concerne une composition cosmétique détergente des fibres kératiniques, en particulier des fibres kératiniques humaines telles que les cheveux, comprenant :
- au moins un tensioactif anionique (A) comportant au moins un groupe sulfate et/ou sulfonate et/ou phosphate,
- au moins un tensioactif anionique carboxylique (B) différent du tensioactif anionique cité en (A),
- au moins un tensioactif amphotère et/ou zwittérionique (C),
- au moins un tensioactif non ionique alkyl(poly)glycoside (D),
- au moins un corps gras non siliconés (E),
- et éventuellement au moins un polymère cationique (F),

le rapport pondéral de la quantité dudit ou desdits tensioactifs anioniques (A) sur la quantité dudit ou desdits tensioactifs non ioniques (D) allant de 0,5 à 1.

## Description

La présente invention concerne de nouvelles compositions de lavage et de conditionnement des matières kératiniques, en particulier des fibres kératiniques humaines telles que les cheveux, comprenant un ou plusieurs corps gras. L'invention concerne aussi leur utilisation pour traiter et laver lesdites matières kératiniques, en particulier lesdites fibres kératiniques, notamment comme shampooing conditionneur.

Dans le domaine des shampoings conditionneurs, on associe généralement une base lavante, à un agent de conditionnement hydrophile (type polymères cationiques, amphotères...) et un agent de conditionnement insoluble (type silicone, huile synthétique ou naturelle, corps gras ou leurs mélanges).

Les agents de conditionnement insolubles, en particulier les corps gras, sont connus et utilisés pour améliorer le démêlage et la douceur des cheveux mouillés et séchés.

Leur utilisation est fortement limitée :
- d'une part, du fait des difficultés de stabilisation dans des compositions détergentes, si on veut maintenir le niveau des qualités d'usage (stabilité, pouvoir moussant, démarrage de mousse),
- d'autre part, du fait des défauts cosmétiques en termes d'alourdissement, de charge et de regraissage liés à des dispersions grossières ou hétérogènes dans ces mêmes compositions détergentes.

Ainsi, il subsiste le besoin de disposer de compositions détergentes contenant des composés gras (très finement dispersés voire solubilisés) qui conduisent à des performances cosmétiques de haut niveau en termes de démêlage et de lissage, sans charge ni alourdissement de la chevelure et en maintenant le niveau des qualités d'usage.

La demanderesse a maintenant découvert qu'il est possible de formuler de telles compositions détergentes, en associant une base tensioactive spécifique à quatre composants, un ou plusieurs corps gras non siliconés, en présence éventuellement d'un ou plusieurs polymères cationiques.

L'invention a donc pour objet une composition de lavage et de conditionnement des matières kératiniques, en particulier des fibres kératiniques, comprenant, de préférence dans un milieu cosmétiquement acceptable, au moins quatre tensioactifs différents définis ci-après, un ou plusieurs corps gras non siliconés, et éventuellement un ou plusieurs polymères cationiques.

Il a été observé que la composition de l'invention confère d'excellentes propriétés cosmétiques à la fois sur cheveux secs et humides, notamment en termes de démêlage, de souplesse, de lissage, de brillance et de discipline des cheveux.

D'autres caractéristiques, aspects, objets et avantages de la présente invention apparaîtront encore plus clairement à la lecture de la description et des exemples qui suivent.

Les différents objets de l'invention vont maintenant être détaillés. L'ensemble des significations et définitions des composés utilisés dans la présente invention données ci-dessous sont valables pour l'ensemble des objets de l'invention.

Dans la présente demande, on qualifie une entité comme étant "anionique" lorsqu'elle possède au moins une charge négative permanente ou lorsqu'elle peut être ionisée en une entité chargée négativement, dans les conditions d'utilisation des compositions de l'invention (milieu, pH par exemple) et ne comprenant pas de charge cationique.

De même, on qualifie une entité comme étant "cationique" lorsqu'elle possède au moins une charge positive ou lorsqu'elle peut être ionisée en une entité chargée positivement, dans les conditions d'utilisation des compositions de l'invention (milieu, pH par exemple) et ne comprenant pas de charge anionique.

On qualifie une entité comme étant "non ionique" lorsqu'elle n'est ni cationique ni anionique au sens de la présente demande, en particulier elle ne comporte aucun groupe cationique ou anionique au sens de la présente demande.

Par "cosmétiquement acceptable" ou "physiologiquement acceptable", on entend qui est compatible avec l'application sur le corps d'un être vivant, en particulier le corps humain, et notamment son cuir chevelu et ses cheveux.

Par simplification, on pourra utiliser les termes :
- "tensioactif (A)" ou "composé (A)" pour désigner un tensioactif anionique comportant dans sa structure un ou plusieurs groupes sulfate et/ou sulfonate et/ou phosphate, utilisé selon l'invention et tel que décrit ultérieurement ;
- "tensioactif (B)" ou "composé (B)" pour un tensioactif anionique alkyl éther carboxylique différent du tensioactif (A), utilisé selon l'invention et tel que décrit ultérieurement ;
- "tensioactif (C)" ou "composé (C)" pour un tensioactif amphotérique et/ou zwittérionique utilisé selon l'invention et tel que décrit ultérieurement ;
- "tensioactif (D)" ou "composé (D)" pour un tensioactif non ionique alkyl(poly)glycoside utilisé selon l'invention et tel que décrit ultérieurement ;
- "composé (E)" pour un corps gras, minéral ou organique, non siliconé utilisé selon l'invention et tel que décrit ultérieurement ;
- "polymère (F)" ou "composé (F)" pour un polymère cationique utilisé selon l'invention et tel que décrit ultérieurement.

Bien entendu, les composés (A), (B), (C), (D), (E) et (F) tels que définis dans les différents objets de l'invention, sont chacun différents les uns des autres.

A défaut d'indication contraire, chacun des composés, optionnels ou non, utilisés ou envisagés dans le cadre de la présente invention peut être présent seul ou en mélange.

La composition cosmétique de lavage et de conditionnement des matières kératiniques, en particulier des fibres kératiniques humaines telles que les cheveux conforme à l'invention, comprend :
- un ou plusieurs tensioactifs anioniques (A) comportant dans leur structure un ou plusieurs groupes sulfate et/ou sulfonate et/ou phosphate,
- un ou plusieurs tensioactifs anioniques alkyl éther carboxylique (B) différents des tensioactifs anioniques cités en (A),
- un ou plusieurs tensioactifs amphotères et/ou zwittérioniques (C),
- un ou plusieurs tensioactifs non ioniques alkyl(poly)glycoside (D),
- un ou plusieurs corps gras non siliconés (E),
- et éventuellement un ou plusieurs polymères cationiques (F),
   le rapport pondéral de la quantité dudit ou desdits tensioactifs anioniques (A) sur la quantité dudit ou desdits tensioactifs non ioniques (D) allant de 0,5 à 1.

### Tensioactif(s) anionique(s) (A) comportant dans leur structure un ou plusieurs groupes sulfate et/ou sulfonate et/ou phosphate.

De préférence, le ou les tensioactifs (A) utilisés selon l'invention ne comprennent pas dans leur structure de groupes carboxyliques (COOH) ou de groupes carboxyliques sous forme de sel (COO⁻).

Le ou les tensioactifs (A) peuvent être oxyéthylénés et/ou oxyproprylénés. Le nombre moyen total de groupements oxyde d'éthylène (OE) et/ou oxyde de propylène (OP) peut alors varier de 2 à 50 et notamment de 2 à 10.

Le ou les tensioactifs (A) utilisables selon l'invention, seul(s)
ou en mélange(s), peuvent être choisis parmi les alkylsulfates, les alkylamidosulfates, les alkyléthersulfates, les alkylamidoéthersulfates, les alkylaryléthersulfates, les alkyléthersulfosuccinates, les acyl iséthionates, les méthyl acyl taurates, et leurs sels ; le groupe alkyle
ou acyle de tous ces différents composés comportant de préférence de 8 à 24 atomes de carbone, et le groupe aryle désignant de préférence un groupement phényle ou benzyle.

Parmi les tensioactifs (A), on préfère utiliser un ou plusieurs tensioactifs anioniques sulfatés, préférentiellement choisi parmi les alkyléthersulfates en C₈-C₁₄ et plus particulièrement ceux en C₁₂-C₁₄.

De préférence, le ou les tensioactifs (A) sont sous forme de sels, et en particulier de sels alcalins notamment de sels de sodium, de sels d'ammonium, de sels d'amines dont les sels d'aminoalcools, et/ou de sels de magnésium. Ces sels comprennent de préférence de 2 à 5 groupements d'oxyde d'éthylène. On utilise de manière plus préférée parmi ceux-ci, les alkyl(C₁₂-C₁₄)sulfates de sodium, de triéthanolamine, de magnésium ou d'ammonium, et/ou les alkyl(C₁₂-C₁₄)éthersulfates de sodium, d'ammonium ou de magnésium, oxyéthylénés, par exemple à 2,2 moles d'oxyde d'éthylène. Mieux encore, le ou les tensioactifs (A) sont choisis parmi les alkyl(C₁₂-C₁₄)éthersulfates de sodium, d'ammonium ou de magnésium, oxyéthylénés à 2,2 moles d'oxyde d'éthylène, tels que commercialisés sous la dénomination TEXAPON N702 par la société COGNIS.

Parmi tous ces tensioactifs anioniques (A), on préfère utiliser les lauryls éther sulfates de sodium ou d'ammonium.

Le ou les tensioactifs (A) sont présents de préférence à raison de 1 % à 50 % en poids, en particulier de 2 % à 25 % en poids, et mieux encore de 3 % à 20 % en poids, par rapport au poids total de la composition.

### Tensioactif(s) anionique(s) alkyl éther carboxylique (B)

Le ou les tensioactifs anioniques alkyl éther carboxylique utilisés selon l'invention comportent de préférence une chaîne alkyle en C₆-C₂₄.

Le ou les tensioactifs (B) utilisés dans le cadre de la présente invention peuvent être choisis, seul(s) ou en mélange(s), parmi :
- les acides alkyl(C₆-C₂₄) éther carboxyliques,
- les acides alkyl(C₆-C₂₄) aryl éther carboxyliques,
- les acides alkyl(C₆-C₂₄) amido éther carboxyliques,
- et leurs sels.

Le ou les tensioactifs (B) utilisés dans le cadre de la présente invention peuvent être oxyalkylénés, de préférence oxyéthylénés et/ou oxypropylénés. Le nombre moyen total de groupements oxyde d'alkylène varie alors de préférence de 2 à 50, en particulier de 2 à 24, et mieux encore de 2 à 15.

Lorsque le ou les tensioactifs (B) sont oxyalkylénés, ils comportent de préférence de 2 à 50 groupements oxyde d'alkylène, et en particulier de 2 à 50 groupement oxyde d'éthylène (OE).

De préférence, le ou les tensioactifs (B) sont neutralisés par un
ou plusieurs sels. Les sels sont en particulier choisis parmi les sels alcalins notamment de sodium, les sels d'ammonium, les sels d'amines dont les sels d'aminoalcools tels que les sels de triéthanolamine ou de monoéthanolamine, et les sels de magnésium.

De manière plus préférée, on utilise les tensioactifs anioniques carboxyliques polyéthoxylés qui répondent à la formule (I) suivante :

R₁(OC₂H₄)ₙOCH₂COOA (1)

dans laquelle :
R₁ représente un groupe ou un mélange de groupes alkyle ou alcényle, linéaire ou ramifié, en C₈-C₂₂, un groupe alkyl(C₈-C₉)phényle, un groupe R₂CONH-CH₂-CH₂- avec R₂ désignant un groupe alkyle ou alcényle, linéaire ou ramifié, en C₁₁-C₂₁,
n est un nombre entier ou décimal (valeur moyenne) pouvant varier de 2 à 24 et de préférence de 2 à 10,
A désigne H, NH₄, Na, K, Li, Mg ou un reste monoéthanolamine ou triéthanolamine. On peut également utiliser des mélanges de composés de formule (I) en particulier des mélanges dans lesquels les groupements R₁ sont différents.

De préférence, R₁ désigne un groupe ou un mélange de groupes choisis parmi les groupes alkyle en C₁₂-C₁₄, cocoyl, oléyl, nonylphényle, et octylphényle ; A désigne un atome d'hydrogène ou de sodium ; et n varie de 2 à 20, et de préférence 2 à 10.

Plus préférentiellement encore, on utilise des composés de formule (1) dans laquelle R₁ désigne un groupe alkyle en C₁₂ ; A désigne un atome d'hydrogène ou de sodium ; et n varie de 2 à 10. Parmi les produits commerciaux, on peut utiliser de préférence les produits vendus par la société CHEM Y sous les dénominations :
AKYPO® NP 70 (R₁=nonylphényle, n=7, A=H)
AKYPO® NP 40 (R₁=nonylphényle, n=4, A=H)
AKYPO®OP 40 (R₁=octylphényle, n=4, A=H)
AKYPO® OP 80 (R₁=octylphényle, n=8, A=H)
AKYPO® OP 190 (R₁=octylphényle, n=19, A=H)
AKYPO® RLM 38 (R₁= alkyle en C₁₂-C₁₄, n=4, A=H)
AKYPO® RLM 38 NV (R₁= alkyle en C₁₂-C₁₄, n=4, A=Na)
AKYPO® RLM 45 CA (R₁= alkyle en C₁₂-C₁₄, n=4,5, A=H)
AKYPO® RLM 45 NV (R₁= alkyle en C₁₂-C₁₄, n=4,5, A=Na)
AKYPO® RLM 100 (R₁= alkyle en C₁₂-C₁₄, n=10, A=H)
AKYPO® RLM 100 NV (R₁= alkyle en C₁₂-C₁₄, n=10, A=Na)
AKYPO® RLM 130 (R₁= alkyle en C₁₂-C₁₄, n=13, A=H)
AKYPO® RLM 160 NV (R₁= alkyle en C₁₂-C₁₄, n=16, A=Na),
   ou par la société SANDOZ sous les dénominations :
   SANDOPAN DTC-Acid (R₁= alkyle en C₁₃, n=6, A=H)
   SANDOPAN DTC (R₁= alkyle en C₁₃, n=6, A=Na)
   SANDOPAN LS 24 (R₁= alkyle en C₁₂-C₁₄, n=12, A=Na)
   SANDOPAN JA 36 (R₁= alkyle en C₁₃, n=18, A=H),
      et plus particulièrement, les produits vendus sous les dénominations suivantes :
      AKYPO® RLM 45 (INCI : Laureth-5 carboxylic acid)
      AKYPO®RLM 100
      AKYPO® RLM 38.

Parmi tous ces tensioactifs anioniques carboxyliques (B), on utilise de préférence les lauryls éther carboxylates de sodium.

Le ou les tensioactifs (B) sont présents de préférence à raison de 0,5 % à 15 % en poids, en particulier de 1 % à 10 % en poids, et mieux encore de 1,5 % à 8 % en poids, par rapport au poids total de la composition.

### Tensioactif(s) amphotère(s) et/ou zwittérionique(s) (C)

Le ou les tensioactifs amphotères et/ou zwittérioniques peuvent être notamment (liste non limitative) des dérivés d'amines secondaires
ou tertiaires aliphatiques, dans lesquels le ou les substituants aliphatiques de la fonction amine secondaire ou tertiaire sont choisis parmi les chaînes linéaires ou ramifiées comportant 8 à 22 atomes de carbone et contenant au moins un groupe anionique hydrosolubilisant tel que par exemple un groupe carboxylate, sulfonate, sulfate, phosphate et/ou phosphonate ; et/ou des bétaïnes.

Parmi les dérivés d'amines utilisables dans le cadre de la présente invention, on peut citer, seuls ou en mélanges, les produits décrits dans les brevets US-2 528 378 et US-2 781 354 et répondant à l'une des structures de formule (II) ou (III) suivante :

- formule (II) : R₂ -CONHCH₂CH₂-N⁺(R₃)(R₄)(CH₂COO⁻) (II)

dans laquelle :
R₂-CO désigne un groupe ou un mélange de groupes choisis parmi les groupes acyle en C₆-C₂₄ ; par exemple la partie acyle en C₆-C₂₄ correspondant à un des acides gras de formule R₂-COOH présents dans l'huile de coprah hydrolysée, un groupe octoyle, un groupe décoyle, un groupe dodécanoyle, ou un mélange de ces groupes,
R₃ désigne un groupement bêta-hydroxyéthyle,
R₄ désigne un groupement carboxyméthyle.

   - formule (III) : R_{2'}-CONHCH₂CH₂-N(B)(C) (III)

   dans laquelle :
   B représente -CH₂CH₂OX',
   C représente -(CH₂)_{z} -Y', avec z = 1 ou 2,
   X' désigne le groupement -CH₂CH₂-COOH ou un atome d'hydrogène,
   Y' désigne -COOH ou le groupe -CH₂-CHOH-SO₃H,
   R_{2'}-CO désigne un groupe acyle en C₆-C₂₄ ; par exemple la partie acyle en C₆-C₂₄ correspondant à un des acides gras de formule R₂-COOH présents dans l'huile de coprah hydrolysée ou l'huile de lin, un groupe octoyle, un groupe décoyle, un groupe dodécanoyle, un groupe stéaroyle, un groupe isostéaroyle, un groupe oléoyle, ou un mélange de ces groupes.

Ces composés sont classés dans le dictionnaire CTFA, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple, on peut citer le Disodium Cocoamphodiacetate commercialisé sous la dénomination commerciale MIRANOL® C2M concentré par la société RHODIA CHIMIE.

Parmi les bétaïnes utilisables dans le cadre de la présente invention, on peut citer les alkyl(C₈-C₂₀) bétaïnes, les sulfobétaïnes, les alkyl(C₈-C₂₀) amidoalkyl(C₁-C₆) bétaïnes et les alkyl(C₈-C₂₀) amidoalkyl(C₁-C₆) sulfobétaïnes, éventuellement hydroxylées, seules ou en mélanges.

De préférence, le ou les tensioactifs amphotériques et/ou zwittérioniques employés dans la présente invention sont choisis parmi les bétaïnes ci-dessus et leurs mélanges. On préfère plus particulièrement utiliser les alkyl(C₈-C₂₀)bétaïnes en particulier la cocobétaïne commercialisée par la société COGNIS sous la dénomination DEHYTON AB 30 en solution aqueuse à 30 % en poids de Matière Active (MA) par rapport au poids total de la solution ; les alkyl(C₈-C₂₀) amidoalkyl(C₁-C₆) bétaïnes, en particulier la cocamidopropylbétaine telle que commercialisée sous la dénomination TEGOBETAINE® F50 par la société GOLDSCHMIDT.

Avantageusement, le ou les tensioactif(s) (C) sont choisis parmi les bétaïnes.

Parmi tous ces tensioactifs amphotères et/ou zwittérioniques (C), on utilise de préférence la cocoamidopropyl bétaïne ou la cocobétaïne.

Le ou les tensioactifs (C) sont présents dans des quantités allant de préférence de 0,1 % à 20 % en poids, en particulier de 1 % à 15 % en poids, et mieux encore de 2 % à 10 % en poids, par rapport au poids total de la composition.

### Tensioactif(s) non ionique(s) alkyl(poly)glycoside (D)

Par "alky(poly)glycoside", on désigne un alkylpolyglycoside ou un alkylmonoglycoside aussi appelé alkylglycoside dans la présente demande, pouvant être alkoxylé par un ou plusieurs groupes oxyde d'alkylène préférentiellement en C₂-C₄.

Le ou les tensioactifs non ioniques alkyl(poly)glycoside utilisés, seul(s) ou en mélange(s), conformément à la présente invention peuvent être représentés par la formule (IV) suivante :

R₁O-(R₂O)ₜ (G)ᵥ (IV)

dans laquelle :
R₁ représente un groupe alkyle saturé ou insaturé, linéaire ou ramifié, comportant environ de 8 à 24 atomes de carbone, un groupe alkylphényle dont le radical alkyle linéaire ou ramifié comporte de 8 à 24 atomes de carbone,
R₂ représente un groupe alkylène comportant environ de 2 à 4 atomes de carbone,
G représente un motif saccharidique comportant de 5 à 6 atomes de carbone,
t désigne une valeur allant de 0 à 10, de préférence 0 à 4, et
v désigne une valeur allant de 1 à 15.

De préférence, le ou les tensioactifs non ioniques alkyl(poly)glycoside répondent à la formule (IV) dans laquelle :
R₁ désigne un groupe alkyle saturé ou insaturé, linéaire ou ramifié, comportant de 8 à 18 atomes de carbone,
G désigne le glucose, le fructose ou le galactose, et de préférence le glucose,
t désigne une valeur allant de 0 à 3, et est de préférence égale à 0,
et R₂ et v sont tels que définis précédemment.

Le degré de polymérisation du ou des tensioactifs non ioniques alkyl(poly)glycoside, tel que représenté par exemple par l'indice v dans la formule (IV), varie en moyenne de 1 à 15, et de préférence de 1 à 4. Ce degré de polymérisation varie plus particulièrement de 1 à 2, et mieux encore de 1,1 à 1,5, en moyenne.

Les liaisons glycosidiques entre les motifs saccharidiques sont en 1,6 ou en 1,4 ; et de préférence en 1,4.

Les composés de formule (IV) pouvant être utilisés dans la présente invention sont notamment représentés par les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société BASF sous la dénomination LUTENSOL GD 70 ou encore ceux vendus par la société CHEM Y sous la dénomination AG10 LK.

On peut également utiliser l'alkyl(C₈-C₁₆) polyglucoside en 1,4, par exemple en solution aqueuse à 53 % en poids du poids total de la solution, commercialisé par COGNIS sous la référence PLANTACARE® 818 UP.

Parmi tous ces tensioactifs non ioniques alkyl(poly)glycosides (D), on utilise de préférence le cocoylpolyglucoside (INCI : Coco-Glucoside) commercialisé par COGNIS sous la référence PLANTACARE® 818.

Le ou les tensioactifs (D) sont présents dans des quantités allant de préférence de 0,1 % à 20 % en poids, en particulier de 1 % à 15 % en poids, et mieux encore de 2 % à 10 % en poids, par rapport au poids total de la composition.

La quantité minimale de tensioactifs anionique(s), amphotérique(s) et/ou zwittérionique(s), et non ionique(s) est celle suffisante pour conférer à la composition un pouvoir moussant et/ou détergent satisfaisant. Des quantités trop importantes de tensioactifs n'apportent pas vraiment d'avantages supplémentaires.

Ainsi, selon l'invention, la quantité totale de tensioactifs anionique(s), amphotérique(s) et/ou zwittérionique(s), et non ionique(s) peut représenter de 4 % à 50 % en poids, de préférence de 6 % à 35 % en poids, et plus préférentiellement de 8 % à 25 % en poids, du poids total de la composition.

Le rapport pondéral de la quantité de tensioactif(s) (A) sur la quantité de tensioactif(s) (B) va de préférence de 0,1 à 10, et en particulier de 0,5 à 5.

Le rapport pondéral de la quantité de tensioactif(s) (A) sur la quantité de tensioactif(s) (C) va de préférence de 0,1 à 10, et en particulier de 0,5 à 5 et mieux encore de 0,7 à 2.

Le rapport pondéral de la quantité de tensioactif(s) anionique(s) (B) sur la quantité de tensioactif(s) (C) va de préférence de 0,1 à 10, et en particulier de 0,2 à 5.

### Corps gras non siliconé (E)

Le ou les corps gras non siliconés (E) utilisés selon l'invention peuvent être solides ou liquides.

Par « corps gras solide non siliconé», on entend au sens de la présente invention un composé organique présentant dans sa structure au moins une chaîne carbonée constituée d'au moins 6 atomes de carbone et ne comportant pas de silicium dans sa structure, ledit composé étant solide et insoluble dans l'eau à température ambiante (25°C) et à pression atmosphérique, ayant un point de fusion supérieur ou égal à 35°C et/ou présentant une viscosité à la température de 40°C et sous un taux de cisaillement de 1s⁻¹ supérieur ou égal à 1 Pa.s.

Par composé « insoluble dans l'eau », on entend un composé qui présente une solubilité dans l'eau inférieure à 1 % en poids et de préférence inférieure à 0,5 % en poids. Le corps gras solide est de préférence soluble à température ambiante (25°C) et à pression atmosphérique dans au moins un solvant organique (par exemple l'éthanol, le chloroforme, ou le benzène) à au moins 1 % en poids.

Les corps gras solides non siliconés selon l'invention peuvent être cristallisés, amorphes ou pâteux.

Le point de fusion va de préférence de 35°C à 250°C, et plus préférentiellement de 40 à 150°C. Ces corps gras solides ont une viscosité à la température de 40 °C et sous un taux de cisaillement de 1s⁻¹, allant de 1 Pa.s. à 1000000 Pa.s. et de préférence de 10 à 1000 Pa.s.

Les mesures de viscosités peuvent réalisées à une température d'environ 40 °C, sur un Carri-Med CSL2-500.

Les points de fusion peuvent être mesurés par DSC ou sur un banc Kofler. Le point de fusion peut être mesuré par analyse calorimétrique différentielle (DSC) avec une vitesse de montée en température de 10 °C par minute. Le point de fusion est alors la température correspondant au sommet du pic endotherme de fusion obtenu lors de la mesure.

Les corps gras solides non siliconés présentant un point de fusion supérieur ou égal à 35 °C sont notamment choisis parmi les alcools gras, les esters gras, les acides gras, les cires sans fonction amide d'origine animale, végétale, minérale ou marine et les cires avec fonction(s) amide(s) telles que les céramides et dérivés de céramides.

Les alcools gras selon l'invention sont de préférence linéaires et saturés, et comportent de 12 à 40 atomes de carbone.

Les alcools gras présentent de préférence la structure ROH, dans laquelle R désigne de préférence un groupement alkyle en C₁₂-C₂₄. R peut être substitué par un ou plusieurs groupements hydroxy et de préférence non substitué.

A titre d'exemple, on peut citer l'alcool myristique, l'alcool cétylique, l'alcool stéarylique, l'alcool béhénique et leurs mélanges.

L'alcool gras peut représenter un mélange d'alcools gras, ce qui signifie que dans un produit commercial peuvent coexister plusieurs espèces d'alcools gras, sous forme d'un mélange.

Les alcools gras de l'invention sont non oxyalkylénés et/ou non glycérolés. Ces alcools gras peuvent être des constituants des cires animales ou végétales.

Les esters gras sont des esters comportant au moins 10 atomes de carbone et de préférence des esters d'un acide carboxylique comportant au moins 10 atomes de carbone et d'un monoalcool ou d'un polyol. Les esters gras selon l'invention peuvent être des mono-, des di- ou des tri-esters.

Les acides carboxyliques ont de préférence de 10 à 30 atomes de carbone et plus particulièrement de 12 à 24 atomes de carbone. Les alcools ont de préférence de 10 à 30 atomes de carbone et plus particulièrement de 12 à 24 atomes de carbone. De préférence, les esters gras solides selon l'invention sont des esters d'un acide gras monocarboxylique comportant au moins 10 atomes de carbone et d'un monoalcool comportant au moins 10 atomes de carbone.

A titre d'esters selon l'invention, on peut citer le myristate de cétyle, le myristate de myristyle, le palmitate de palmityle, le palmitate de stéaryle, le stéarate de palmityle, le stéarate de stéaryle, et leurs mélanges.

Les esters gras peuvent être des constituants des cires animales
ou végétales.

Les acides gras ont de préférence de 10 à 30 atomes de carbone et plus particulièrement de 12 à 24 atomes de carbone. A titre d'acides gras selon l'invention, on peut citer l'acide stéarique, l'acide béhénique, l'acide laurique, et leur mélange.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque.

A titre de cires sans fonction amide utilisables dans la présente invention, on peut citer les cires d'origine animale telles que le spermaceti, la cire de lanoline et les dérivés de lanoline, les cires d'abeilles et les cires d'abeilles modifiées (cerabellina) ; les cires végétales telles que la cire de Carnauba, de Candellila, d'Alfa, d'Ouricury, du Japon, la cire d'olivier, la cire de riz, la cire de jojoba hydrogénée ou les cires absolues de fleurs telles que la cire essentielle de fleur de cassis vendue par la Société BERTIN (France), le beurre de karité, le beurre de cacao ou les cires de fibres de liège ou de canne à sucre ; les cires minérales, par exemple, de paraffine, de vaseline, de lignite, les cires microcristallines et les ozokérites ; d'autres cires ou matières premières cireuses utilisables selon l'invention sont notamment les cires marines telles que celle vendue par la Société SOPHIM sous la référence M82, et leurs mélanges.

Selon la présente invention, on entend par « céramide ou dérivé de céramide », un composé naturel ou synthétique, choisi parmi les céramides, les glycocéramides, les pseudocéramides et les néocéramides.

De tels composés sont par exemple décrits dans les demandes de brevet DE 4 424 530, DE 4 424 533, DE 4 402 929, DE 4 420 736, W0 95/23807, EP-A-0646572, W0 95/16665, FR 2 673 179, EP-A-0 227 994 et WO 94/07844, W0 94/24097, W0 94/10131 dont les enseignements sont ici inclus à titre de référence.

Les cires à fonction(s) amide(s) dont les céramides ou dérivés de céramides utilisables selon la présente invention répondent par exemple à la formule générale (V) suivante : 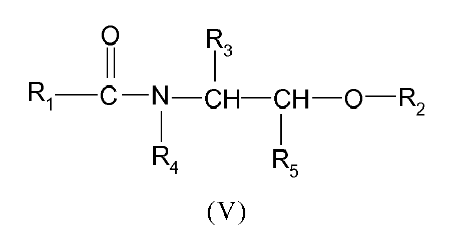 dans laquelle :
- R₁ désigne :

- soit un groupe hydrocarboné, linéaire ou ramifié, saturé ou insaturé, en C₁-C₅₀, de préférence en C₅-C₅₀, ce groupe pouvant être substitué par un ou plusieurs groupements hydroxyle éventuellement estérifié par un acide R₇COOH, R₇ étant un groupe hydrocarboné, saturé ou insaturé, linéaire ou ramifié, éventuellement mono ou polyhydroxylé, en C₁-C₃₅, le ou les hydroxyles du groupe R₇ pouvant être estérifié par un acide gras saturé ou insaturé, linéaire ou ramifié, éventuellement mono ou polyhydroxylé, en C₁-C₃₅ ;
- soit un groupe R"-(NR-CO)-R', où R désigne un atome d'hydrogène ou un groupe hydrocarboné C₁-C₂₀ mono ou polyhydroxylé, préférentiellement monohydroxylé, R' et R" sont des groupes hydrocarbonés dont la somme des atomes de carbone va de 9 à 30, R' étant un groupe divalent.
- soit un groupe R₈-O-CO-(CH₂)ₚ, où R₈ désigne un groupe hydrocarboné en C₁-C₂₀, p est un entier variant de 1 à 12.
- R₂ est choisi parmi un atome d'hydrogène, un groupe de type saccharidique, en particulier un groupe (glycosyle)ₙ, (galactosyle)ₘ ou sulfogalactosyle, un résidu de sulfate ou de phosphate, un groupe phosphoryléthylamine et un groupe phosphoryléthylammonium, dans lesquels n est un entier variant de 1 à 4 et m est un entier variant de 1 à 8 ;
- R₃ désigne un atome d'hydrogène ou un groupe hydrocarboné en C₁-C₃₃, saturé ou insaturé, mono ou polyhydroxylé ou non hydroxylé, le ou les hydroxyles pouvant être estérifiés par un acide minéral ou un acide R₇COOH, R₇ ayant les mêmes significations que ci-dessus, le ou les hydroxyles pouvant être éthérifiés par un groupe (glycosyle)ₙ, (galactosyle)ₘ, sulfogalactosyle, phosphoryléthylamine
ou phosphoryléthylammonium dans lesquels n et m sont définis ci-avant, R₃ pouvant également être substitué par un ou plusieurs groupes alkyle en C₁-C₁₄ ; de préférence, R₃ désigne un groupe alpha-hydroxyalkyle en C₁₅-C₂₆, le groupement hydroxyle étant éventuellement estérifié par un alpha-hydroxyacide en C₁₆-C₃₀ ;
- R₄ désigne un atome d'hydrogène, un groupe méthyle, éthyle, un groupe hydrocarboné en C₃-C₅₀, saturé ou insaturé, linéaire ou ramifié, éventuellement hydroxylé ou un groupe -CH₂-CHOH-CH₂-OR₆ dans lequel R₆ désigne un groupe hydrocarboné en C₁₀-C₂₆ ou un radical R₈-O-CO-(CH₂)ₚ, R₈ désigne un groupe hydrocarboné en C₁-C₂₀, p est un entier variant de 1 à 12,
- R₅ désigne un atome d'hydrogène ou un groupe hydrocarboné en C₁-C₃₀ saturé ou insaturé, linéaire ou ramifié, éventuellement mono
ou polyhydroxylé, le ou les hydroxyles pouvant être éthérifiés par un groupe (glycosyle)ₙ, (galactosyle)ₘ, sulfogalactosyle, phosphoryléthylamine ou phosphoryléthylammonium dans lesquels n et m sont définis ci-avant, sous réserve que lorsque R₃ et R₅ désignent hydrogène ou lorsque R₃ désigne hydrogène et R₅ désigne méthyle alors R₄ ne désigne pas un atome d'hydrogène, un groupe méthyle ou éthyle.

Parmi les composés de formule (V), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179, dont les enseignements sont ici inclus à titre de référence.

Les céramides et dérivés de céramide plus particulièrement préférés selon l'invention sont les composés de formule (V) pour lesquels R₁ désigne un alkyle saturé ou insaturé dérivé d'acides gras en C₁₄-C₂₂ éventuellement hydroxyle ; R₂ désigne un atome d'hydrogène ; et R₃ désigne un groupe linéaire en C₁₁-C₁₇ éventuellement hydroxyle et de préférence en C₁₃-C₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4-triol et en particulier la N-stéaroyl phytosphingosine,
- le 2-N-palmitoylamino-hexadécane-1,3-diol
- ou les mélanges de ces composés.

On peut également utiliser les composés de formule (V) pour lesquels R₁ désigne un groupe alkyle saturé ou insaturé dérivé d'acides gras en C₁₂-C₂₂ ; R₂ désigne un groupe galactosyle ou sulfogalactosyle ; et R₃ désigne un groupe hydrocarboné en C₁₂-C₂₂, saturé ou insaturé et de préférence un groupement CH(OH)-CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les céramides et dérivés de céramides dont les formules sont représentées dans les demandes de brevet EP-A-0227994 et WO 94/07844.

De tels composés sont par exemple le QUESTAMIDE H (bis-(N-hydroxyéthyl N-cétyl) malonamide) vendu par la société QUEST.

On peut également utiliser le N-docosanoyl N-méthyl-D-glucamine décrit dans la demande de brevet W0 94/24097.

De préférence, le corps gras solide non siliconé n'est pas un acide gras.

Parmi tous ces corps gras solides non siliconés, on préfère utiliser la 2-oléamido-1,3-octadécanediol et/ou la cire de Carnauba.

Par « corps gras liquide non siliconé », on entend au sens de la présente invention un composé organique présentant dans sa structure au moins une chaîne carbonée constituée d'au moins 6 atomes de carbone et ne comportant pas d'atomes de silicium dans sa structure et qui, à température ambiante (25°C) et à pression atmosphérique, est un liquide insoluble dans l'eau (c'est-à-dire, présente une solubilité dans l'eau inférieure à 1 % en poids et de préférence inférieure à 0,5 % en poids), et est soluble, dans les mêmes conditions de température et de pression, dans au moins un solvant organique (par exemple l'éthanol, le chloroforme, ou le benzène) à au moins 1 % en poids.

De préférence, le ou les corps gras liquides non siliconés sont choisis parmi :
- les esters gras liquides, en particulier les triglycérides parmi lesquels on citera les huiles végétales.
- les huiles hydrocarbonées,
- les acides gras liquides, en particulier ceux en C₈-C₃₀ présentant des chaînes carbonées ramifiées ou possédant une ou plusieurs (de préférence 1 à 3) insaturations,
- les alcools gras liquides, en particulier ceux en C₈-C₃₀ présentant des chaînes carbonées ramifiées ou possédant une ou plusieurs (de préférence 1 à 3) insaturations.

Les esters gras liquides sont des esters liquides d'acides carboxyliques et d'alcools, l'un au moins de l'acide ou de l'alcool comportant au moins 8 atomes de carbone et plus particulièrement de 10 à 40 atomes de carbone. Les acides et les alcools à l'origine des esters peuvent être linéaires ou ramifiés et saturés ou insaturés (avec 1 à 3 insaturations). L'alcool peut être un monoalcool ou un polyol. L'acide peut être éventuellement mono ou polyhydroxylé.

A titre d'exemple d'esters d'acide gras utilisables, on peut citer l'octanoate de stéaryle (huile de Purcellin®), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le lactate de 2-octyldodécyle, le néopentanoate d'isostéaryle, le néopentanoate de tridécyle, le néopentanoate d'isocétyle et le néopentanoate d'isoarachidyle, et leurs mélanges.

Les huiles végétales peuvent être choisies parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot, l'huile de cade, la cire liquide de jojoba, l'huile de calophyllum et leurs mélanges.

Les huiles hydrocarbonées sont de préférence des alcanes non alkoxylés comportant au moins 8 atomes de carbone.

Comme huiles hydrocarbonées, on peut citer les huiles minérales notamment l'huile de paraffine et l'huile de vaseline, les isoparaffines tels que les polyisobutylènes, les polydécènes ; et leurs mélanges.

Les acides gras de l'invention sont de préférence en C₁₀-C₂₂. Ils sont de préférence ramifiés et/ou comportent 1 à 3 insaturations. On peut citer en particulier l'acide isostéarique et l'acide oléique.

Les alcools gras selon l'invention sont de préférence ramifiés et/ou insaturés, et comportent de 12 à 40 atomes de carbone. Les alcools gras de l'invention sont non oxyalkylénés.

Les alcools gras présentent de préférence la structure R-OH,
dans laquelle R désigne de préférence un groupement alkyle ramifié en C₁₂-C₂₄ ou alkényle en C₁₂-C₂₄. R peut être substitué par un ou plusieurs groupements hydroxy. De préférence, R ne contient pas de groupement hydroxy.

A titre d'exemple on peut citer l'alcool oléique, l'alcool isocétylique, l'alcool isostéarylique, le 2-octyl-1-dodécanol, le 2-éthylhexyl dodécanol, et leur(s) mélange(s).

Les alcools gras, les acides gras ou les esters gras peuvent être des mélanges, ce qui signifie que dans un produit commercial peuvent coexister plusieurs espèces notamment de longueurs de chaînes différentes, sous forme d'un mélange.

Parmi toutes ces familles de corps gras liquides non siliconés, on préfère utiliser des esters gras liquides et/ou des huiles hydrocarbonées, en particulier des huiles végétales, et plus préférentiellement l'huile d'avocat, les isoparaffines, l'huile d'abricot et/ou le myristate d'isopropyle.

Le ou les corps gras non siliconés peuvent être présents dans la composition à une teneur allant de 0,05 % à 8 % en poids, de préférence de 0,1 % à 5 % en poids, et mieux encore de 0,5 % à 3 % en poids, par rapport au poids total de la composition.

### Polymère(s) cationique(s) (F)

Lorsque la composition selon l'invention comprend un ou plusieurs polymères cationiques, ceux-ci peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, FR-A-2 383 660, FR-A-2 598 611, FR-A-2 470 596, FR-A-2 519 863 et FR-A-2875 503.

Les polymères cationiques préférés sont choisis parmi ceux qui contiennent dans leur structure des motifs comportant des groupements amine primaires, secondaires, tertiaires et/ou quaternaires pouvant par exemple soit faire partie de la chaîne principale polymère, soit être portés par un substituant latéral directement relié à celle-ci.

Parmi les polymères cationiques, on peut citer plus particulièrement les polymères de la famille des polyamines, polyaminoamides et polyammoniums quaternaires. Parmi ces polymères, on peut citer :
(1) Les homopolymères ou copolymères dérivés d'esters ou d'amides acryliques ou méthacryliques, réticulés ou non, et comportant au moins un des motifs de formules (VI), (VII), (VIII) ou (IX) suivantes : 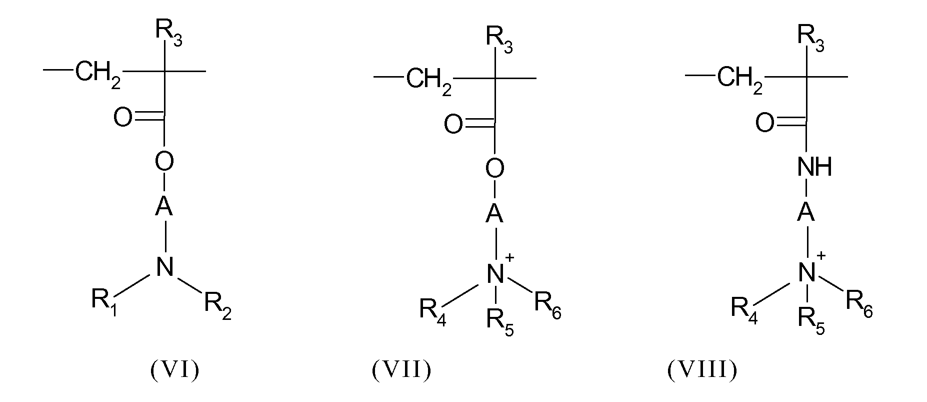 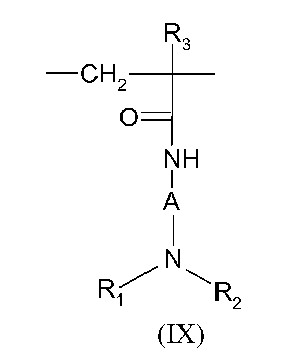 dans lesquelles
   R₁ et R₂, identiques ou différents, représentent chacun un atome d'hydrogène ou un groupe alkyle ayant de 1 à 6 atomes de carbone et de préférence méthyle ou éthyle ;
   R₃, identiques ou différents, désignent chacun un atome d'hydrogène ou un groupe CH₃ ;
   A, identiques ou différents, représentent chacun un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent chacun un groupe alkyle ayant de 1 à 6 atomes de carbone ou un groupe benzyle et de préférence un groupe alkyle ayant de 1 à 6 atomes de carbone ;
   X⁻ désigne un anion dérivé d'un acide minéral ou organique tel qu'un anion méthosulfate ou un halogénure tel que chlorure ou bromure.

Les polymères de la famille (1) peuvent contenir en outre un ou plusieurs motif(s) dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.

Ainsi, parmi ces polymères de la famille (1), on peut citer :
- les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
- les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2 077 143 et 2 393 573,
- les terpolymères méthacrylate de diméthyl amino éthyle / vinylcaprolactame / vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- les copolymères vinylpyrrolidone / méthacrylamide de diméthylaminopropyle quaternisés tel que le produit vendu sous la dénomination "GAFQUAT HS 100" par la société ISP,
- les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide / chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société CIBA.

(2) Les polysaccharides cationiques notamment choisis parmi :
   a) les dérivés d'éther de cellulose comportant des groupements ammonium quaternaire décrits dans le brevet français 1 492 597, et en particulier les polymères commercialisés sous les dénominations « JR » (JR 400, JR 125, JR 30M) ou « LR » (LR 400, LR 30M) par la société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
   b) les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl-celluloses greffées notamment avec un sel de méthacryloyléthyl triméthylammonium, de méthacrylmidopropyl triméthylammonium ou de diméthyl-diallylammonium.
      Les produits commercialisés répondant à cette définition sont plus particulièrement les produits vendus sous la dénomination « Celquat L 200 » et « Celquat H 100 » par la société National Starch.
   c) les gommes de guar contenant des groupements cationiques trialkylammonium. On utilise par exemple des gommes de guar modifiées par un sel (par exemple, un sel de chlorure) de 2,3-époxypropyl triméthylammonium.
      De tels produits sont commercialisés notamment sous les dénominations commerciales de JAGUAR C13 S, JAGUAR C 15, JAGUAR C 17 ou JAGUAR C162 par la société MEYHALL.
(3) Les polymères constitués de motifs pipérazinyle et de radicaux divalents alkylène ou hydroxyalkylène à chaîne droite ou ramifiée, éventuellement interrompue par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(4) Les polyaminoamides cationiques solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; ces polyaminoamides peuvent être alcoylés ou s'ils comportent une ou plusieurs fonctions amines tertiaires, quaternisées. De tels polymères sont notamment décrits dans les brevets français 2 252 840 et 2 368 508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylamino hydroxyalcoyldialcoylène triamine dans lesquels le groupe alcoyle comporte de 1 à 4 atomes de carbone et désigne de préférence méthyle, éthyle, propyle. De tels polymères sont notamment décrits dans le brevet français 1 583 363.
   Parmi ces dérivés, on peut citer plus particulièrement les polymères acide adipique / diméthylaminohydroxypropyl / diéthylène triamine vendus sous la dénomination "Cartaretine F, F4 ou F8" par la société Sandoz.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique / époxypropyl / diéthylène-triamine.
(7) Les cyclopolymères d'alkyl diallyl amine ou de dialkyl diallyl ammonium tels que les homopolymères ou copolymères comportant comme constituant principal de la chaîne des motifs répondant aux formules (Xₐ) ou (X_{b}) : 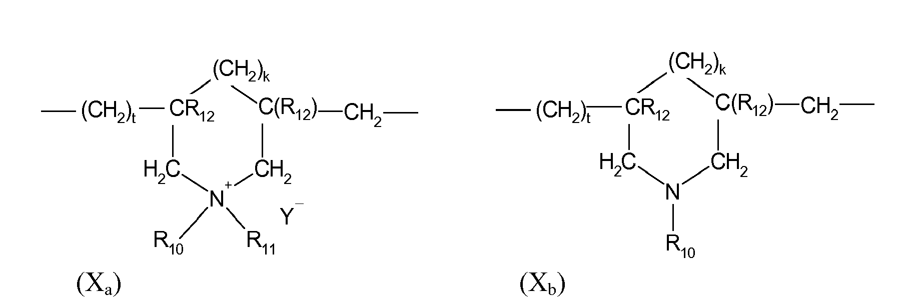 dans lesquelles k et t sont égaux à 0 ou 1, la somme k + t étant égale à 1 ; R₁₂ désigne un atome d'hydrogène ou un groupe méthyle ; R₁₀ et R₁₁, désignent chacun, indépendamment l'un de l'autre, un groupement alkyle ayant de 1 à 6 atomes de carbone, un groupement hydroxyalkyle dans lequel le groupement alkyle a de préférence de 1 à 5 atomes de carbone, un groupement amidoalkyle inférieur (i.e. dont la partie alkyle est en C₁-C₄), ou alors R₁₀ et R₁₁ peuvent désigner conjointement avec l'atome d'azote auquel ils sont rattachés, un groupement hétérocyclique, tel que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.

De préférence, R₁₀ et R₁₁ désignent chacun, indépendamment l'un de l'autre, un groupement alkyle ayant de 1 à 4 atomes de carbone.

Parmi les polymères définis ci-dessus, on peut citer les homopolymères de chlorure de dialkyldiallylammonium, plus particulièrement l'homopolymère de chlorure de diméthyldiallylammonium (nom INCI : Polyquaternium-6) vendu sous la dénomination "MERQUAT® 100" par la société NALCO (et ses homologues de faibles masses moléculaires moyenne en poids) et les copolymères de chlorure de dialkyldiallylammonium, plus particulièrement le copolymère de chlorure de diméthyldiallylammonium et d'acrylamide commercialisés sous la dénomination "MERQUAT® 550".
(8) Le polymère de diammonium quaternaire contenant des motifs récurrents répondant à la formule (XI) : 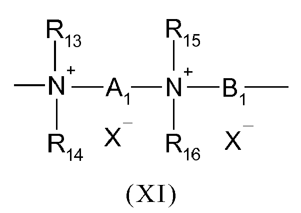 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs (i.e. dont la partie alkyle est en C₁-C₄), ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent chacun un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique ; A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)ₙ-

      dans lequel n désigne un nombre entier de 0 à 7 et E' désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent chacun un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH-.

De préférence, X⁻ est un anion tel que le chlorure ou le bromure.

Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

On peut utiliser plus particulièrement les polymères qui sont constitués de motifs récurrents répondant à la formule (XII) : 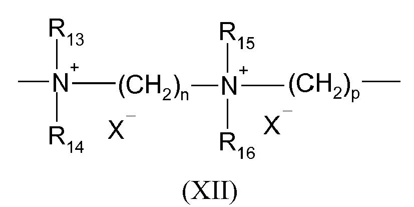 dans laquelle R₁₃, R₁₄, R₁₅ et R₁₆, identiques ou différents, désignent chacun un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique. De préférence, R₁₃, R₁₄, R₁₅ et R₁₆ désignent chacun un groupe méthyle. A titre d'exemple de polymère utilisable répondant à la formule (XII), on peut citer le chlorure d'hexadiméthrine, commercialisé sous la dénomination MEXOMERE PO par la société CHIMEX.
(9) Les polymères de polyammonium quaternaire constitués de motifs de formule (XIII) : 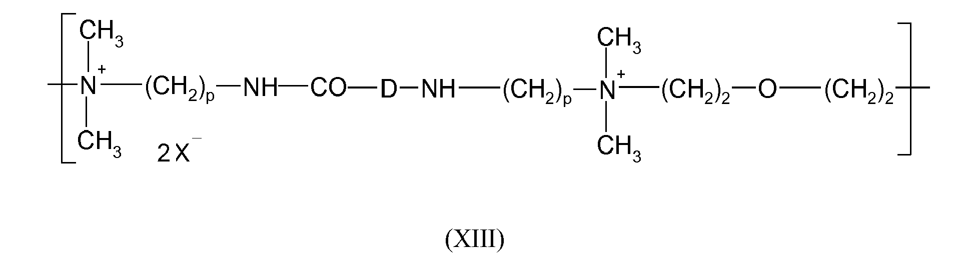 dans laquelle :
   p désigne un nombre entier variant de 1 à 6 environ,
   D peut être nul ou peut représenter un groupement
      -(CH₂)ᵣ -CO- dans lequel r désigne un nombre égal à 4 ou à 7, et
   X⁻ est un anion dérivé d'un acide minéral ou organique.

Les polymères cationiques comportant des motifs de formule (XIII) sont notamment décrits dans la demande de brevet EP-A-122 324 et peuvent être préparés selon les procédés décrits dans les brevets U.S.A. n° 4 157 388, 4 390 689, 4 702 906, 4 719 282.

Parmi ces polymères, on préfère ceux de masse moléculaire mesurée par RMN du Carbone 13 inférieure à 100000, et dans la formule de laquelle :
p est égal à 3, et,
   a) D représente un groupement -(CH₂)₄-CO- , X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1,
   b) D représente un groupement -(CH₂)₇-CO- , X désigne un atome de chlore, la masse moléculaire mesurée par RMN du Carbone 13 (RMN¹³C) étant d'environ 8100 ; un polymère de ce type est proposé par la société MIRANOL sous le nom de MIRAPOL-AZ1,
   c) D désigne la valeur zéro, X désigne un atome de chlore, la masse moléculaire mesurée par RMN du Carbone 13, (RMN¹³C) étant d'environ 25500 ; un polymère de ce type est vendu par la société MIRANOL sous le nom MIRAPOL-A15,
   d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).

Plus particulièrement encore, on préfère selon l'invention le polymère à motifs de formule (XIII) dans laquelle p est égal à 3, D désigne la valeur zéro, X désigne un atome de chlore, la masse moléculaire mesurée par RMN du Carbone 13, (RMN¹³C) étant d'environ 25500.
(10) Les polymères quaternaires de vinylpyrrolidone et de vinylimidazole tels que par exemple les produits commercialisés sous les dénominations Luviquat FC 905, FC 550 et FC 370 par la société B.A.S.F.
(11) Les polyamines cationiques comme le Polyquart H vendu par HENKEL, référencé sous le nom de " POLYETHYLENEGLYCOL (15) TALLOW POLYAMINE " dans le dictionnaire CTFA.
(12) Les homopolymères ou copolymères de vinylamide et en particulier les homopolymères de vinylamide partiellement hydrolysés tels que les poly(vinylamine/vinylamide). Ces polymères sont formés à partir d'au moins un monomère vinylamide répondant à la formule suivante :

   H₂C=CR²NRC(O)R¹

   dans laquelle R, R¹ et R² sont chacun choisis parmi un atome d'hydrogène, un groupe alkyle en C₁-C₂₀, un groupe aryle et un groupe alkylaryle dont la partie alkyle comprend de 1 à 20 atomes de carbone.

En particulier, ledit monomère peut être choisi parmi le N-vinylformamide, le N-méthyl-N-vinylacétamide et le N-vinyl acétamide. De préférence, on utilise le poly(vinylamine/N-vinylformamide) tel que commercialisé sous la dénomination CATIOFAST VMP par la société BASF ou sous la dénomination LUPAMIN 9030 par la société BASF.

Ces polymères peuvent être formés par exemple par polymérisation radicalaire d'un monomère vinylamide puis hydrolyse acide ou basique partielle des fonctions amides en fonctions amines quaternisables, tel que décrit dans les demandes WO 2007/005577, US 5,374,334, US 6,426,383 et US 6,894,110.
(13) Les polyuréthanes constitués essentiellement :
   (a1) d'au moins un motif cationique dérivé d'au moins une amine tertiaire ou quaternaire présentant au moins deux fonctions réactives à hydrogène labile,
   (a2) d'au moins un motif non ionique dérivé d'au moins une polyoléfine présentant au moins deux fonctions réactives à hydrogène labile, ladite polyoléfine comprenant au moins 10 % en moles d'unités comprenant au moins une double liaison C=C (carbone-carbone), par rapport à la totalité des unités formant ladite polyoléfine ;
   (b) d'au moins un motif dérivé d'un composé comportant au moins deux fonctions isocyanate.

Le polymère selon l'invention présente de préférence un caractère élastique ; on entend par là que ledit polymère est un matériau macromoléculaire qui retourne rapidement à sa forme et à ses dimensions initiales après cessation d'une contrainte faible ayant produit une déformation importante.

Ces polymères peuvent être obtenus par polycondensation des composés portant des fonctions réactives à hydrogène labile avec des composés comportant au moins deux fonctions isocyanate ;

On entend par fonctions réactives à hydrogène labile des fonctions capables, après départ d'un atome d'hydrogène, de former des liaisons covalentes avec les fonctions isocyanate des composés comportant au moins deux fonctions isocyanate. On peut citer à titre d'exemple de telles fonctions les groupes hydroxyle, amine primaire, amine secondaire, ou encore les groupes thiol.

Selon la nature des fonctions réactives portant l'hydrogène labile (-OH, -NH₂, -NHR ou -SH), la polycondensation conduit à, respectivement, des polyuréthanes, des polyurées ou des polythiouréthanes. Ainsi, les polymères utilisables dans les compositions selon l'invention peuvent être des copolymères uréthane/urée et/ou thiouréthane. Tous ces polymères sont regroupés dans la présente demande, par souci de simplification, sous le terme de polyuréthanes.

Le ou les polyuréthanes cationiques utilisables dans la composition selon l'invention comprennent donc au moins un motif cationique (a1) résultant d'au moins une amine tertiaire ou quaternaire présentant au moins deux fonctions réactives à hydrogène labile.

L'amine tertiaire est de préférence protonable à un pH choisi entre pH 1 et pH 12. Par "protonable", on entend que ladite fonction amine tertiaire peut être neutralisée au moins partiellement par un agent neutralisant ou par une fonction du milieu dans lequel il est formulé.

Lorsque les amines tertiaires ou quaternaires formant les motifs (a1) portent plus de deux fonctions à hydrogène labile, les polyuréthanes obtenus présentent une structure ramifiée.

Toutefois, de préférence, les amines tertiaires ou quaternaires formant les motifs (a1) ne présentent que deux fonctions réactives à hydrogène labile et les polyuréthanes obtenus par polycondensation ont par conséquent une structure essentiellement linéaire.

Il est bien entendu également possible d'utiliser un mélange d'amines difonctionnelles contenant, ou non, une faible proportion d'amines portant plus de deux fonctions réactives à hydrogène labile.

Les amines tertiaires ou quaternaires formant les motifs cationiques (a1) sont de préférence choisies parmi les composés correspondant à l'une ou plusieurs des formules suivantes : 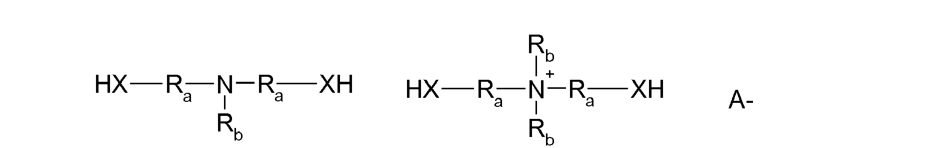 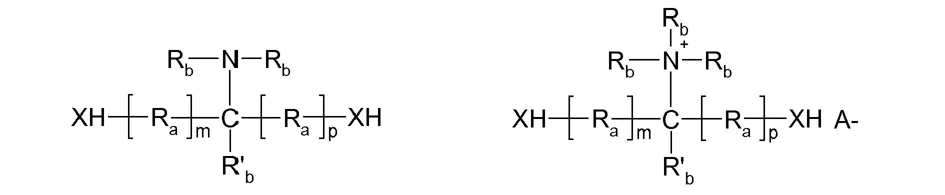 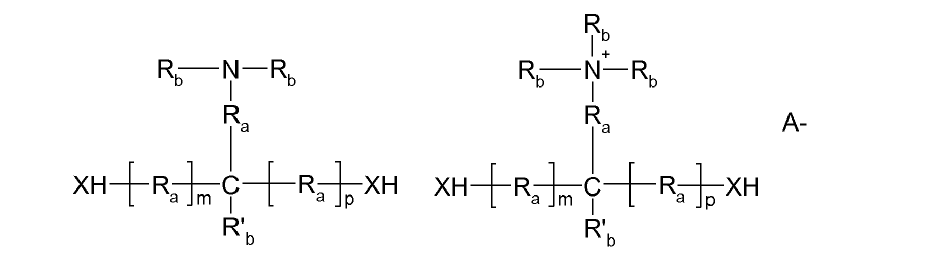 dans lesquelles :
- chaque Rₐ, indépendamment les uns des autres, représente un groupe divalent alkylène en C₁-C₆, linéaire ou ramifié, ou bien cycloalkylène en C₃-C₆ ou arylène, ou leurs mélanges ; ces groupes pouvant être substitués par un ou plusieurs atomes d'halogène et/ou comporter un ou plusieurs hétéroatomes choisis parmi O, N, P et S,
- chaque R_{b} représente indépendamment les uns des autres, un groupe alkyle linéaire ou ramifié, en C₁-C₆, ou bien cycloalkyle en C₃-C₆ ou encore aryle, ou leurs mélanges ; ces groupes pouvant être substitués par un ou plusieurs atomes d'halogène et/ou comporter un ou plusieurs hétéroatomes choisis parmi O, N, P et S,
- chaque R'_{b} représente un atome d'hydrogène ou un groupe alkyle linéaire ou ramifié, en C₁-C₆, ou bien cycloalkyle en C₃-C₆ ou encore aryle, ou leurs mélanges ; ces groupes pouvant être substitués par un ou plusieurs atomes d'halogène et/ou comporter un ou plusieurs hétéroatomes choisis parmi O, N, P et S,
- m et p sont, indépendamment l'un de l'autre, égaux à 0 ou 1 ; de préférence m= 1 et p= 1 ;
- chaque X représente, indépendamment les uns des autres, un atome d'oxygène ou de soufre, ou un groupe NH ou NR_{c}, où R_{c} représente un groupe alkyle en C₁-C₆, et
- A⁻ représente un contre-ion physiologiquement acceptable, et notamment un halogénure tel que chlorure ou bromure.

De préférence, les amines sont choisies parmi les composés répondant à l'une ou plusieurs des formules : 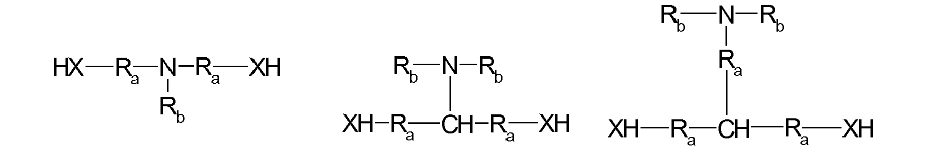 dans lesquelles :
- Rₐ est un groupe divalent alkylène en C₁-C₆, linéaire ou ramifié, notamment méthylène ou éthylène ; et/ou
- R_{b} est un groupe alkyle linéaire ou ramifié, en C₁-C₆, notamment un groupe méthyle, éthyle, n-butyle, isobutyle ou tert-butyle ; et/ou
- X désigne un atome d'oxygène.

Encore plus préférentiellement, les amines sont de formule : 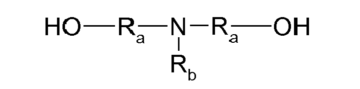 dans laquelle Rₐ est un groupe divalent alkylène en C₁-C₆, linéaire ou ramifié, notamment méthylène ou éthylène ; et R_{b} est un groupe alkyle linéaire ou ramifié, en C₁-C₆, notamment un groupe méthyle, éthyle, n-butyle, isobutyle ou tert-butyle.

On peut citer à titre d'amines tertiaires particulièrement préférées, la N-méthyldiéthanolamine et la N-tert-butyldiéthanolamine.

Les amines tertiaires, protonables, peuvent être neutralisées, totalement ou partiellement, par un agent neutralisant, de type acide organique comprenant au moins une fonction acide carboxylique, sulfonique et/ou phosphorique ou par un acide minéral. On peut citer à titre d'exemple d'acides préférés l'acide chlorhydrique, l'acide sulfurique, l'acide acétique, l'acide propionique, l'acide citrique, l'acide gluconique, l'acide tartrique, l'acide lactique, l'acide phosphorique, l'acide benzoïque, l'acide stéarique, l'acide oléique, l'acide 2-éthylcaproïque, l'acide béhénique, le chlorhydrate de bétaïne, et leurs mélanges.

Le ou les polyuréthanes cationiques utilisables dans la composition selon l'invention comprennent également au moins un motif non ionique (a2) résultant d'au moins une polyoléfine présentant au moins deux fonctions réactives à hydrogène labile, ladite polyoléfine comprenant au moins 10 % en mole d'unités comprenant au moins une double liaison C=C (résiduelle), par rapport à la totalité des unités formant ladite polyoléfine.

De préférence, la ou les polyoléfines sont non ioniques.

De préférence, les fonctions réactives à hydrogène labile sont situées aux extrémités de la polyoléfine. Notamment, lesdites fonctions réactives à hydrogène labile sont des hydroxydes. De façon préférentielle, le nombre de motifs hydroxyde est proche de, voire égal à, 2.

De préférence encore, la ou les polyoléfines formant le motif (a2) est choisie parmi les homopolymères et/ou copolymères d'oléfines, portant à leurs extrémités des fonctions réactives à hydrogène labile et ayant une température de transition vitreuse (Tg), mesurée par analyse enthalpique différentielle (DSC, differential scanning calorimetry) selon la norme ASTM D3418-97, inférieure à 10°C.

Le ou les polyuréthanes dans la composition selon l'invention peuvent comprendre plusieurs motifs (a2) résultant de plusieurs polyoléfines, identiques ou différentes (mélanges de polyoléfines) ; toutefois, dans ce cas, chacune des polyoléfines comprend au moins 10 % molaire d'unités comprenant au moins une double liaison C=C.

On entend par « unité » comprenant une double liaison C=C, une unité qui comprend au moins une double liaison C=C résiduelle, de préférence une seule double liaison ; il peut s'agir, par exemple d'une unité issue de la polymérisation d'une unité butadiène ou isoprène, toutes formes isomériques incluses (cis ou trans, 1,2 ou 1,4).

La polyoléfine utilisable peut être un homopolymère d'oléfines.

On peut par exemple citer les homopolymères de 1,2-butadiène, de 1,4-butadiène ou d'isoprène, et notamment :
- les 1,4-polybutadiène, sous leurs formes cis et trans : 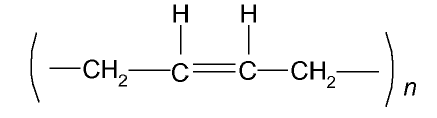
- les 1,2-polybutadiène :

   -[CH₂-CH(CH=CH₂)-]ₙ
- les poly(cis-1,4-isoprène) : 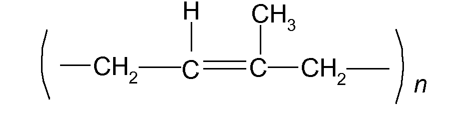
- les poly(trans-1,4-isoprène) :

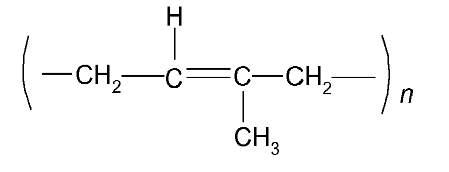

La polyoléfine utilisable peut également être un copolymère de différentes oléfines (copolymère d'oléfines), sous réserve que la polyoléfine finale comprenne au moins 10 % molaire d'unités comprenant au moins une double liaison C=C.

Dans un premier mode de réalisation, ladite polyoléfine peut être exclusivement constituée d'unités comprenant au moins une double liaison C=C. On peut par exemple citer les copolymères, notamment statistiques, comprenant des unités 1,2-butadiène et/ou des unités 1,4-butadiène dans ses formes cis et/ou trans, et/ou des unités isoprène, notamment cis-1,4-isoprène et trans-1,4-isoprène, en mélange. On peut notamment citer les copolymères statistiques (1,2-butadiène/1,4-butadiène).

De préférence, la ou les polyoléfines utilisables peuvent être statistiques et à terminaisons hydroxy et répondre à la structure suivante : 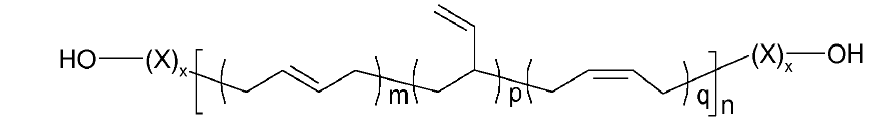 dans laquelle :
m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
n est un entier allant de 10 à 100, notamment de 15 à 50 ;
x=0 ou 1, et
X représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.

Elles peuvent présenter, de préférence, une masse moléculaire moyenne en nombre, Mn, allant de 400 à 50000, de préférence de 500 à 30000, en particulier de 1000 à 15000, et encore mieux de 1500 à 12000.

Plus particulièrement, on peut citer :
- les polybutadiènes à terminaisons hydroxy, tels que les polymères de structure : 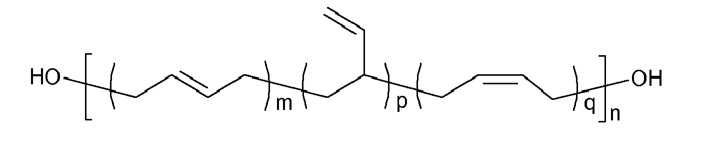 avec m=0,6, p=0,2 et q=0,2 (fractions molaires) et n=25.

On peut en particulier citer les produits commerciaux « Poly bd R20LM » et « Poly bd R45HTLO » de Sartomer.
- les polybutadiènes à terminaisons primaires hydroxy, tels que les polymères pouvant être représentés par la structure suivante : 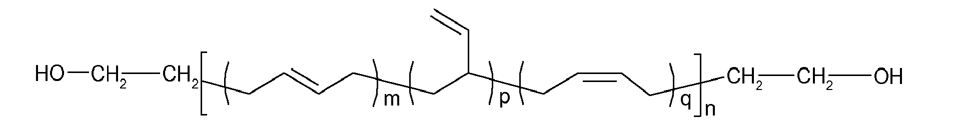 qui sont des copolymères statistiques notamment de 1,4-cis-butadiène et de 1,4-trans-butadiène, avec m=0,17, p=0,65 et q=0,18 (fractions molaires) et n est tel que le poids moléculaire moyen en nombre Mn varie de 1000 à 10000, notamment de 2000 à 6000 (g.mol⁻¹)_{.}

On peut en particulier citer les produits commerciaux KRASOL LBH-P 2000, 3000 ou 5000 de Sartomer.
- les polybutadiènes à terminaisons secondaires hydroxy, tels que les polymères pouvant être représentés par la structure suivante : 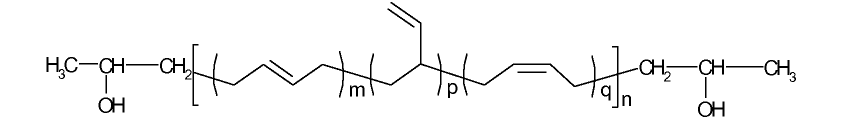 qui sont des copolymères statistiques de 1,4-cis-butadiène et de 1,4-trans-butadiène, avec m=0,17, p=0,65 et q=0,18 (fractions molaires), et n est tel que le poids moléculaire moyen en nombre Mn varie de 1000 à 12000, notamment de 2000 à 10000 (g.mol⁻¹).

On peut en particulier citer les produits commerciaux KRASOL LBH 2000, 3000, 5000 ou 10000 de Sartomer.

Dans un second mode de réalisation, ladite ou lesdites polyoléfines peuvent comprendre en outre des unités additionnelles ne comprenant pas de double liaison C=C.

Toutefois, ces unités additionnelles sont présentes en une quantité maximale de 90 % molaire, étant donné que la polyoléfine finale doit comprendre au moins 10 % molaire d'unités comprenant au moins une double liaison C=C.

Ces unités oléfines additionnelles peuvent notamment être choisies parmi les unités éthylène -(CH₂-CH₂)ₙ-, propylène -(CH₂-CH₂-CH₂)ₙ-, isopropylène -(CH₂CH(CH₃))ₙ-, et/ou butylène de formule : 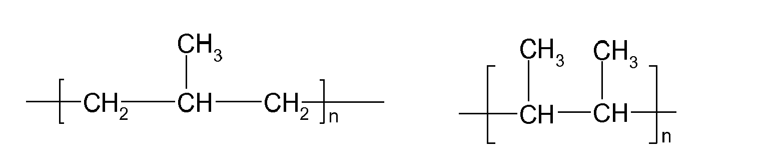 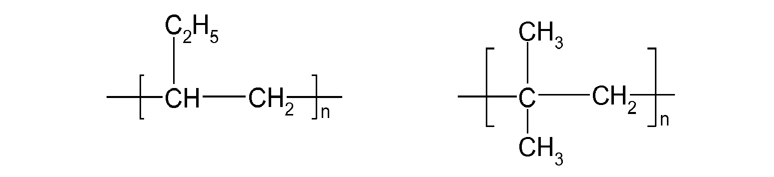 ainsi que leurs mélanges.

Les homopolymères ou copolymères d'oléfines telles que définies ci-dessus peuvent subir, ultérieurement à la polymérisation, une hydrogénation partielle des doubles liaisons résiduelles. Cette hydrogénation ne peut en aucun cas être totale.

En effet, la ou les polyoléfines susceptibles d'être employées pour former les motifs (a2) selon l'invention doivent obligatoirement comprendre au moins 10 % en mole d'unités comprenant au moins une double liaison C=C (résiduelle), par rapport à la totalité des unités formant ladite polyoléfine.

Elles comprennent de préférence au moins 20 % molaire, notamment au moins 40 % molaire, voire au moins 50 % molaire, préférentiellement au moins 80 % molaire, et tout particulièrement 100 % molaire, d'unités comprenant au moins une double liaison C=C, notamment comprenant une seule double liaison C=C.

Cette teneur en unité comportant au moins une double liaison C=C peut notamment être déterminée par les techniques usuelles, notamment par RMN ou par dosage à l'iode.

De préférence, la ou les polyoléfines utilisables pour former les motifs non ioniques (a2) ont une masse moléculaire en nombre (Mn) allant de 400 à 50000, de préférence de 500 à 30000, en particulier de 1000 à 15000, et encore mieux de 1500 à 12000.

De préférence, la ou les polyoléfines susceptibles d'être utilisées dans le cadre de l'invention sont :
- des homopolymères tels que le 1,4-polybutadiène et le 1,2-polybutadiène ;
- des copolymères de structure : 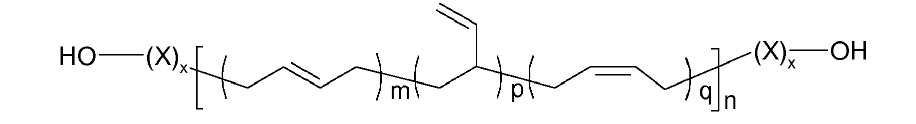 dans laquelle :
   m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
   n est un entier allant de 10 à 100, notamment de 15 à 50 ;
   x=0 ou 1, et
   X représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.

Le ou les polyuréthanes cationiques utilisables dans la composition selon l'invention comprend également au moins un motif (b) résultant d'au moins un composé comportant au moins deux fonctions isocyanate.

Il peut bien évidemment s'agir d'un mélange de plusieurs composés comportant au moins deux fonctions isocyanate.

Les composés comportant au moins deux fonctions isocyanate peuvent être choisis parmi les diisocyanates, ou les mélanges d'un diisocyanate et d'un polyisocyanate comportant plus de deux fonctions isocyanates, ledit polyisocyanate représentant de préférence 0,1 % à 40 % en poids dudit mélange, notamment 0,5 % à 35 % en poids, voire 1 % à 30 % en poids, du poids dudit mélange.

Les composés comportant au moins deux fonctions isocyanate peuvent de préférence être choisis parmi les diisocyanates aliphatiques, cycliques conjugués ou non, aromatiques ou non. Ils peuvent notamment être choisis parmi le méthylènediphényldiisocyanate, le méthylènecyclohexanediisocyanate, l'isophorone diisocyanate, le toluène diisocyanate, le naphtalène diisocyanate, le 1,4-butanediisocyanate et le 1,6-hexane-diisocyanate, et leur mélange ; de préférence l'isophorone diisocyanate.

Préférentiellement, le ou les polyuréthanes utilisables dans la composition selon l'invention sont constitués essentiellement :
- d'au moins un motif cationique résultant d'amines de formule : 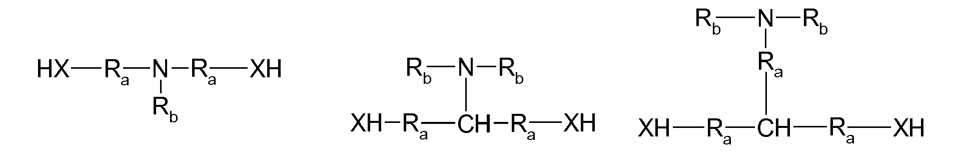 dans lesquelles :
   Rₐ est un groupe divalent alkylène en C₁-C₆, linéaire ou ramifié, notamment un groupe méthylène ou éthylène ;
   R_{b} est un groupe alkyle linéaire ou ramifié, en C₁-C₆, notamment un groupe méthyle, éthyle, n-butyle, isobutyle ou tert-butyle ;
   et X représente un atome d'hydrogène.
- d'au moins un motif non ionique résultant de polyoléfines choisies parmi les homopolymères 1,4-polybutadiène et 1,2-polybutadiène ; ou les copolymères de structure : 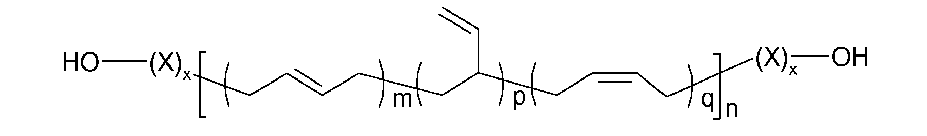 dans laquelle :
   m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
   n est un entier allant de 10 à 100, notamment de 15 à 50 ;
   x=0 ou 1, et
   X représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.
- d'au moins un motif résultant de diisocyanates aliphatiques.

Encore plus préférentiellement, le ou les polyuréthanes utilisables selon l'invention sont constitués essentiellement :
- d'au moins un motif cationique résultant d'amines de formule : 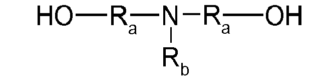 dans laquelle Rₐ est un groupe divalent alkylène en C₁-C₆, linéaire ou ramifié, notamment un groupe méthylène ou éthylène ; et R_{b} est un groupe alkyle linéaire ou ramifié, en C₁-C₆, notamment un groupe méthyle, éthyle, n-butyle, isobutyle ou tert-butyle ;
   et plus particulièrement d'au moins un motif cationique choisi parmi la N-méthyldiéthanolamine et la N-tert-butyldiéthanolamine ;
   - d'au moins un motif non ionique résultant de polyoléfines de structure : 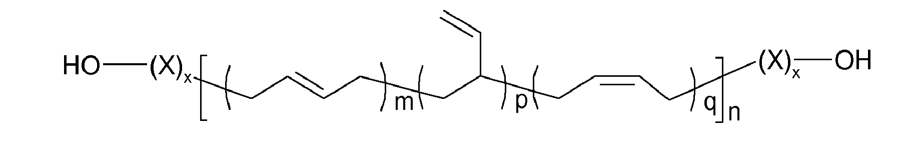 dans laquelle :
      m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
      n est un entier allant de 10 à 100, notamment de 15 à 50 ;
      x=0 ou 1, et
      X = représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.
   - d'au moins un motif résultant de diisocyanates choisis parmi le méthylènecyclohexanediisocyanate, l'isophorone diisocyanate, le 1,4-butanediisocyanate et le 1,6-hexane-diisocyanate ; de préférence l'isophorone diisocyanate.

Les polyuréthanes utilisables selon l'invention sont constitués essentiellement de motifs (a1), (a2) et (b) tels que définis ci-dessus, ce qui implique qu'il ne comprend pas de motifs additionnels autres que ceux-ci.

Parmi tous les polyuréthanes cités ci-dessus, on utilise de préférence, les polyuréthanes formés par les monomères suivants :
(a1) au moins un N-méthyl diéthanolamine (noté NMDEA),
(a2) au moins un copolymère d'éthylène/butylène non ionique tel que commercialisé sous la dénomination Krasol LBH-P 2000, et
(b) au moins un isophrone diisocyanate (noté IPDI).

De préférence, les amines formant les motifs cationiques (a1) représentent de 0,1 % à 50 %, en particulier de 1 % à 30 %, et mieux encore de 5 % à 20 % en poids, du poids total du polyuréthane final.

De préférence, les polyoléfines formant les motifs non ioniques (a2) représentent de 30 % à 99 % en poids, en particulier de 50 % à 90 %, et mieux encore de 60 % à 80 % en poids, du poids total de polyuréthane final.

De préférence, les composés comprenant au moins deux fonctions isocyanate, formant les motifs (b) sont présents en une quantité essentiellement stoechiométrique par rapport à la somme des amines tertiaires/quaternaires formant les motifs (a1) et des polyoléfines formant les motifs (a2).

De préférence, les composés comprenant au moins deux fonctions isocyanate formant les motifs (b) représentent de 1 % à 60 % en poids, en particulier de 5 % à 50 % en poids, et mieux encore de 15 % à 35 % en poids, du poids total du polyuréthane final.

De manière plus préférée, les polyuréthanes selon l'invention sont formés à partir de :
- 20 % à 55 %, notamment de 25 % à 50%, voire de 30 % à 47 % molaire d'amine tertiaire ou quaternaire susceptible de former les motifs (a1) ;
- 1 % à 30 %, notamment de 2 % à 25 %, voire de 3 % à 20 % molaire de polyoléfine susceptible de former les motifs (a2) ; et
- 30 % à 65 %, notamment 35 % à 60 %, voire de 45 % à 55 % molaire de composé comportant au moins deux fonctions isocyanate susceptible de former les motifs (b).

Préférentiellement, le rapport molaire entre (b) et (a1)+(a2) est proche de 1.

Ces polyuréthanes et leurs synthèses sont décrits par exemple dans la demande de brevet FR-A-289 8 603.
(14) D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre, seuls ou en mélanges, un ou plusieurs polymères cationiques choisis parmi :
- les copolymères d'acrylamide et de méthacrylate de diméthyl aminoéthyle quaternisé au sulfate de diméthyle ou avec un halogénure de diméthyle,
   - les copolymères d'acrylamide et de méthosulfate de méthacryloyloxyéthyltriméthylammonium,
   - les copolymères vinylpyrrolidone/acrylate ou méthacrylate de dialkylaminoalkyle quaternisés ou non. Ces polymères sont décrits en détail dans les brevets français 2 077 143 et 2 393 573,
   - les terpolymères méthacrylate de diméthylaminoéthyle/vinylcaprolactame/vinylpyrrolidone,
   - les copolymères vinylpyrrolidone/méthacrylamidopropyl-diméthylamine, et
   - les copolymères vinylpyrrolidone/méthacrylamide de diméthylaminopropyle quaternisé.
- les ammoniums quaternaires d'hydroxyéthylcellulose ayant réagi avec un époxyde substitué par un groupement triméthylammonium (nom INCI : Polyquaternium-10),
- les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium,
- les gommes de guar contenant des groupements cationiques trialkylammonium,
- l'homopolymère de chlorure de diméthyldiallylammonium (nom INCI : Polyquaternium-6) vendu sous la dénomination "MERQUAT® 100" par la société NALCO (et ses homologues de faibles masses moléculaires moyenne en poids),
- le chlorure d'hexadiméthrine,
- les polymères quaternaires de vinylpyrrolidone et de vinylimidazole,
- les poly(vinylamine/vinylamide),
- les polyuréthanes formés par les monomères suivants :
   (a1) au moins un N-méthyl diéthanolamine (noté NMDEA),
   (a2) au moins un copolymère d'éthylène/butylène non ionique tel que commercialisé sous la dénomination Krasol LBH-P 2000, et
   (b) au moins un isophrone diisocyanate (noté IPDI),
- les polyalkylèneimines, en particulier les polyéthylèneimines.

Lorsque le ou les polymères (F) sont présents dans la composition selon l'invention, ils sont présents dans des quantités allant de préférence de 0,01 à 10 % en poids, en particulier de 0,05 à 8 % en poids, et mieux encore de 0,1 à 5 % en poids, par rapport au poids total de la composition.

### Agent bénéfique pour les matières kératiniques

La composition de l'invention peut également contenir en plus un ou plusieurs agents bénéfiques pour les matières kératiniques différents des composés (A), (B), (C), (D), (E) et (F) sus-cités et choisis parmi :
(1) Les saccharides, les oligosaccharides, les polysaccharides additionnels autres que ceux cités précédemment, hydrolysés ou non, modifiés ou non,
(2) Les acides aminés, les oligopeptides, les peptides, les protéines, hydrolysés ou non, modifiés ou non,
(3) Les polyols et les polyéthylènes glycols,
(4) Les émollients,
(5) Les agents hydratants,
(6) Les acides organiques hydroxylés,
(7) Les filtres UV,
(8) Les antioxydants et les agents anti-radicaux libres,
(9) Les chélatants,
(10) Les agents antipelliculaires,
(11) Les agents antialopéciques,
(12) Les agents régulateurs de séborrhée,
(13) Les agents apaisants,
(14) Les tensioactifs cationiques,
(15) Les silicones organomodifiées ou non, volatiles ou non,
(16) Les polymères anioniques,
(17) Les polymères non ioniques,
(18) Les polymères amphotères,
(19) Les pigments,
(20) Les charges minérales ou organiques,
(21) Les argiles,
(22) Les minéraux colloïdaux.

Lorsque le ou les agents bénéfiques sont présents dans la composition de l'invention, ils sont chacun présents dans des quantités allant de préférence de 0,001 % à 10 % en poids, en particulier de 0,01 % à 5 % en poids, et mieux encore de 0,1 % à 3 % en poids, par rapport au poids total de la composition.

La composition détergente selon l'invention présente de préférence un pH allant de 2 à 10. De préférence, ce pH va de 4 à 7. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la 1,3-propanediamine, ou encore par ajout d'un acide minéral ou organique, de préférence un acide polycarboxylique tel que l'acide citrique, l'acide tartrique, l'acide maléique, l'acide succinique, l'acide adipique ou leurs mélanges.

La composition selon l'invention comprend de préférence un milieu cosmétiquement acceptable.

Le milieu cosmétiquement acceptable peut être un milieu aqueux constitué uniquement par de l'eau ou par un mélange d'eau et d'un ou plusieurs solvants cosmétiquement acceptables, tel qu'un alcool inférieur en C₁-C₄, comme l'éthanol, l'isopropanol, le tertiobutanol, le n-butanol ; les alkylèneglycols comme le propylèneglycol, l'hexylèneglycol ; le glycérol ; ou leurs mélanges.

La composition selon l'invention comprend de préférence au moins 30 % en poids d'eau, en particulier de 50 % à 90 % en poids et encore préférentiellement de 70 % à 85 % en poids, par rapport au poids total de la composition.

La composition selon l'invention peut comprendre en plus des composés (A), (B), (C), (D) et (E) tels que définis précédemment, un ou plusieurs additifs cosmétiques.

Par "additif cosmétique", on entend un composé cosmétiquement acceptable différent des composés (A), (B), (C), (D) et (E) utilisés selon l'invention et ajouté dans une composition de l'invention. En particulier, lorsque la composition selon l'invention comprend un ou plusieurs polymères (F) et/ou un ou plusieurs agents bénéfiques pour les matières kératiniques, et comprend en outre un ou plusieurs additifs, ces additifs sont différents desdits polymères (F) et desdits agents bénéfiques.

Parmi les additifs cosmétiques utilisables, on peut citer de façon non exhaustive les adjuvants classiques bien connus dans la technique, tels que les électrolytes non polymériques, les agents réducteurs, les agents oxydants, les colorants directs ou d'oxydation, les agents épaississants polymériques ou non, les acides et les bases organiques ou minérales, les plastifiants, les azurants optiques, les nacres, les agents nacrants, les parfums, les peptisants, les conservateurs, les tensioactifs autres que ceux de l'invention ou leurs mélanges.

Ce ou ces additifs sont chacun présents dans la composition selon l'invention en une quantité allant de préférence de 0 % à 20 % en poids, par rapport au poids total de la composition.

L'homme de métier veillera à choisir le ou les éventuels additifs et leurs quantités de manière à ce qu'ils ne nuisent pas aux propriétés des compositions de la présente invention.

Un autre objet de l'invention est un procédé de traitement cosmétique, **caractérisé en ce qu**'il comprend l'application sur des matières kératiniques, en particulier des fibres kératiniques humaines telles que des cheveux, d'une quantité efficace d'une composition selon l'invention.

La composition peut être appliquée sur cheveux secs ou mouillés, et de préférence sur cheveux mouillés ou humides.

Selon un mode de mise en oeuvre préféré, un tel procédé consiste à appliquer sur les cheveux une quantité efficace de la composition selon l'invention, malaxer éventuellement les cheveux, laisser pauser éventuellement la composition sur les cheveux, et rincer.

Lorsqu'on laisse pauser ladite composition sur les cheveux le temps de pause varie généralement de 0,5 à 5 minutes. La composition est généralement rincée à l'eau.

La présente invention a aussi pour objet l'utilisation d'une composition selon l'invention dans ou pour la fabrication d'une composition cosmétique détergente des fibres kératiniques, en particulier des fibres kératiniques humaines telles que les cheveux, telle que par exemple la fabrication d'un shampooing conditionnant.

Un autre objet de l'invention concerne l'utilisation d'une composition selon l'invention pour traiter et en particulier conditionner des matières kératiniques, en particulier des fibres kératiniques humaines telles que les cheveux.

Les exemples suivants sont donnés à titre illustratif de la présente invention, et ne sauraient en limiter la portée.

### EXEMPLES

On a réalisé les compositions de shampooings suivantes. Sauf indication contraire, les quantités sont indiquées en pour cent en poids de matière active par rapport au poids total de la composition. On note entre parenthèses les quantités de produits commerciaux utilisées, exprimées en pourcentage en poids par rapport au poids total de la composition.

| **Compositions** | **Ex. 1** | **Ex. 2** | **Ex. 3** | **Ex. 4** |
|---|---|---|---|---|
| 2-oléamido-1,3-octadécanediol | 0,5 | 0,3 | - | - |
| Polyisobutène hydrogéné [1] | - | 1 | - | - |
| Cire de Jojoba hydrogénée | - | - | 1 | - |
| Cire de Carnauba | - | - | - | 0,2 |
| Polyquaternium-6 à 40 % en poids dans l'eau [2] | 0,7 | 0,4 | 0,4 | - |
| | (1,75) | (1) | (1) | |
| Polyquaternium-10 à 91 % en poids dans un mélange eau/isopropanol [3] | - | - | 0,7 | 0,7 |
| | | | (0,8) | (0,8) |
| Propylène glycol | 2 | 2 | 2 | 2 |
| Cocobétaïne à 30 % en poids dans | 5,4 | 5,4 | 5,4 | 5,4 |
| l'eau [4] | (18) | (18) | (18) | (18) |
| Chlorure de sodium | 2,5 | 2,5 | - | 2,5 |
| Lauryl éther carboxylate (5OE) de sodium à 90 % en poids dans l'eau [5] | 3 | 3 | 3 | 3 |
| | (3,3) | (3,3) | (3,3) | (3,3) |
| Lauryl éther sulfate (2,2 OE) de sodium à 70 % en poids dans l'eau [6] | 4 | 4 | 4 | 4 |
| | (5,7) | (5,7) | (5,7) | (5,7) |
| Alkyl(C₈-C₁₆) polyglucoside à 53 % en poids dans l'eau [7] | 5 | 5 | 5 | 5 |
| | (9,45) | (9,45) | (9,45) | (9,45) |
| Acide salicylique | 0,2 | 0,2 | 0,2 | 0,2 |
| Para-hydroxybenzoate d'éthyle | 0,15 | 0,15 | 0,15 | 0,15 |
| Benzoate de sodium | 0,5 | 0,5 | 0,5 | 0,5 |
| Para-hydroxybenzoate de méthyle sel | 0,4 | 0,4 | 0,4 | 0,4 |
| de sodium | | | | |
| Parfum | 0,5 | 0,5 | 0,5 | 0,5 |
| Eau | q.s.p. | q.s.p. | q.s.p. | q.s.p. |
| | 100 | 100 | 100 | 100 |

[1] PARLEAM vendu par NOF CORPORATION
[2] MERQUAT 100 vendu par NALCO
[3] UCARE POLYMER JR 400 LT vendu par AMERCHOL
[4] DEHYTON AB 30 vendu par COGNIS
[5] AKYPO RLM 45 vendu par KAO
[6] TEXAPON AOS 225 UP vendu par COGNIS
[7] PLANTACARE 818 UP vendu par COGNIS

| **Compositions** | **Ex. 5** | **Ex. 6** | **Ex. 7** | **Ex. 8** | **Ex. 9** | **Ex. 10** |
|---|---|---|---|---|---|---|
| Huile d'avocat | 0,6 | 1 | 1,5 | - | - | - |
| Polyisobutène hydrogéné [1] | | 0,5 | - | 2 | - | - |
| Huile d'abricot | - | - | - | - | 1 | |
| Myristate d'isopropyle | - | - | - | - | - | 2 |
| Polyquaternium-6 à 40 % en poids dans l'eau [2] | - | - | 0,4 | 0,7 | 0,7 | - |
| | | | (1) | (1,75) | (1,75) | |
| Polyquaternium-10 à 91 % en poids dans un mélange eau/isopropanol [3] | 0,5 | 0,7 | - | - | - | 0,7 |
| | (0,5) | (0,8) | | | | (0,8) |
| Propylène glycol | 2 | 2 | 2 | 2 | 2 | 2 |
| Cocobétaïne à 30 % en poids dans l'eau [4] | 5,4 | 5,4 | 5,4 | 5,4 | 5,4 | 5,4 |
| | (18) | (18) | (18) | (18) | (18) | (18) |
| Ceteareth-60 myristyl glycol [5] | - | - | 2,1 | 2,1 | 2,1 | - |
| Chlorure de sodium | - | - | - | 2,5 | - | - |
| Lauryl éther carboxylate (5 OE) de sodium à 90 % en poids dans l'eau [6] | 3 | 3 | 3 | 3 | 3 | 3 |
| | (3,3) | (3,3) | (3,3) | (3,3) | (3,3) | (3,3) |
| Lauryl éther sulfate (2,2 OE) de sodium à 70 % en poids dans l'eau [7] | 4 | 4 | 4 | 4 | 4 | 4 |
| | (5,7) | (5,7) | (5,7) | (5,7) | (5,7) | (5,7) |
| Alkyl(C₈-C₁₆) polyglucoside à 53 % en poids dans l'eau [8] | 5 | 5 | 5 | 5 | 5 | 5 |
| | (9,45) | (9,45) | (9,45) | (9,45) | (9,45) | (9,45) |
| Acide salicylique | 0,2 | 0,2 | 0,2 | 0,2 | 0,2 | 0,2 |
| Para-hydroxybenzoate d'éthyle | 0,15 | 0,15 | 0,15 | 0,15 | 0,15 | 0,15 |
| Benzoate de sodium | 0,5 | 0,5 | 0,5 | 0,5 | 0,5 | 0,5 |
| Para-hydroxybenzoate de méthyle sel de sodium | 0,4 | 0,4 | 0,4 | 0,4 | 0,4 | 0,4 |
| Parfum | 0,5 | 0,5 | 0,5 | 0,5 | 0,5 | 0,5 |
| Eau | q.s.p. | q.s.p. | q.s.p. | q.s.p. | q.s.p. | q.s.p. |
| | 100 | 100 | 100 | 100 | 100 | 100 |

[1] PARLEAM vendu par NOF CORPORATION
[2] MERQUAT 100 vendu par NALCO
[3] UCARE POLYMER JR 400 LT vendu par AMERCHOL
[4] DEHYTON AB 30 vendu par COGNIS
[5] ELFACOS GT 2825 vendu par AKZO NOBEL
[6] AKYPO RLM 45 vendu par KAO
[7] TEXAPON AOS 225 UP vendu par COGNIS
[8] PLANTACARE 818 UP vendu par COGNIS

### Résultats :

Les compositions des exemples 1 à 10 présentent de bonnes qualités de mousse (démarrage et abondance).

Les cheveux humides sont lisses, souples et se démêlent facilement.

Les cheveux secs sont lisses, disciplinés et brillants.

## Revendications

1. Composition de lavage et de conditionnement des matières kératiniques, en particulier des fibres kératiniques humaines telles que les cheveux, comprenant :
- un ou plusieurs tensioactifs anioniques (A) comportant dans leur structure un ou plusieurs groupes sulfate et/ou sulfonate et/ou phosphate,
- un ou plusieurs tensioactifs anioniques alkyl éther carboxylique (B) différents des tensioactifs anioniques cités en (A),
- un ou plusieurs tensioactifs amphotères et/ou zwittérioniques (C),
- un ou plusieurs tensioactifs non ioniques alkyl(poly)glycoside (D),
- un ou plusieurs corps gras non siliconés (E),
- et éventuellement un ou plusieurs polymères cationiques (F), le rapport pondéral de la quantité dudit ou desdits tensioactifs anioniques (A) sur la quantité dudit ou desdits tensioactifs non ioniques (D) allant de 0,5 à 1.

2. Composition selon la revendication 1, **caractérisée en ce que** le ou les tensioactifs (A) sont choisis, seul(s) ou en mélange(s), parmi les alkylsulfates, les alkylamidosulfates, les alkyléthersulfates, les alkylamidoéthersulfates, les alkylaryléthersulfates, les alkyléthersulfosuccinates, les acyl iséthionates, les méthyl acyl taurates, et leurs sels ; le groupe alkyle ou acyle de tous ces différents composés comportant de préférence de 8 à 24 atomes de carbone, et le groupe aryle désignant de préférence un groupement phényle ou benzyle, éventuellement oxyéthylénés et/ou oxyproprylénés.

3. Composition selon l'une quelconque des revendications précédentes, **caractérisée en ce que** le ou les tensioactifs (A) sont présents à raison de 1 % à 50 % en poids, et de préférence de 2 % à 25 % en poids, par rapport au poids total de la composition.

4. Composition selon l'une quelconque des revendications précédentes, **caractérisée en ce que** le ou les tensioactifs (B) sont polyéthoxylés et répondent à la formule (I) suivante :
R₁(OC₂H₄)ₙOCH₂COOA (I)
dans laquelle :
R₁ représente un groupe ou un mélange de groupes alkyle ou alcényle, linéaire ou ramifié, en C₈-C₂₂, un groupe alkyl(C₈-C₉)phényle, un groupe R₂CONH-CH₂-CH₂- avec R₂ désignant un radical alkyle ou alcényle, linéaire ou ramifié, en C₁₁-C₂₁,
n est un nombre entier ou décimal (valeur moyenne) pouvant varier de 2 à 24 et de préférence de 2 à 10,
A désigne H, NH₄, Na, K, Li, Mg ou un reste monoéthanolamine ou triéthanolamine.

5. Composition selon l'une quelconque des revendications précédentes, **caractérisée en ce que** le ou les tensioactifs (B) sont présents à raison de 0,5 % à 15 % en poids, et de préférence de 1 % à 10 % en poids, par rapport au poids total de la composition.

6. Composition selon l'une quelconque des revendications précédentes, **caractérisée en ce que** le ou les tensioactifs (C) sont choisis parmi les bétaïnes.

7. Composition selon l'une quelconque des revendications précédentes, **caractérisée en ce que** le ou les tensioactifs (C) sont présents à raison de 0,1 % à 20 % en poids, et de préférence de 1 % à 15 % en poids, par rapport au poids total de la composition.

8. Composition selon l'une quelconque des revendications précédentes, **caractérisée en ce que** le ou les tensioactifs (D) sont choisis, seul(s) ou en mélange(s), parmi les composés répondant à la formule (IV) suivante :
R₁O-(R₂O)ₜ (G)ᵥ (IV)
dans laquelle :
R₁ représente un groupe alkyle saturé ou insaturé, linéaire ou ramifié, comportant environ de 8 à 24 atomes de carbone, un groupe alkylphényle dont le radical alkyle linéaire ou ramifié comporte de 8 à 24 atomes de carbone,
R₂ représente un groupe alkylène comportant environ de 2 à 4 atomes de carbone,
G représente un motif saccharidique comportant de 5 à 6 atomes de carbone,
t désigne une valeur allant de 0 à 10, de préférence 0 à 4, et
v désigne une valeur allant de 1 à 15.

9. Composition selon l'une quelconque des revendications précédentes, **caractérisée en ce que** le ou les tensioactifs (D) sont présents à raison de 0,1 % à 25 % en poids, et de préférence de 1 % à 15 % en poids, par rapport au poids total de la composition.

10. Composition selon l'une quelconque des revendications précédentes, **caractérisée en ce que** le ou les corps gras non siliconés (E) sont choisis parmi les corps gras solides non siliconés suivants :
- les corps gras solides sans fonction amide choisis parmi les les cires d'origine animale, végétale, minérale ou marine, les esters gras, les acides gras, les alcools gras,
- les corps gras solides à fonction(s) amide(s) choisis parmi les céramides et dérivés de céramides,
- et leurs mélanges.
et/ou parmi les corps gras liquides non siliconés suivants :
- les esters gras liquides,
- les huiles hydrocarbonées,
- les acides gras liquides,
- les alcools gras liquides,
- et leurs mélanges.

11. Composition selon l'une quelconque des revendications précédentes, **caractérisée en ce que** le ou les corps gras non siliconés (E) sont présents dans des quantités allant de 0,05 % à 8 % en poids, de préférence de 0,1 % à 5 % en poids, par rapport au poids total de la composition.

12. Composition selon l'une quelconque des revendications précédentes, **caractérisée en ce que** le ou les polymères (F) sont présents dans des quantités allant de 0,01 % à 10 % en poids, et de préférence de 0,05 % à 8 % en poids, par rapport au poids total de la composition.

13. Procédé de traitement cosmétique, **caractérisé en ce qu'**il comprend l'application sur des matières kératiniques, en particulier des fibres kératiniques humaines telles que des cheveux, d'une quantité efficace d'une composition selon l'une quelconque des revendications 1 à 12.

14. Utilisation d'une composition telle que définie dans l'une quelconque des revendications 1 à 12 pour laver et traiter des matières kératiniques, en particulier des fibres kératiniques humaines telles que les cheveux.
